# EUROPÄISCHE PATENTSCHRIFT

(11) **EP 0 369 420 B1**
(45) Veröffentlichungstag und Bekanntmachung des Hinweises auf die Patenterteilung: **17.03.1993**
(21) Anmeldenummer: 89121130.2
(22) Anmeldetag: 15.11.1989
(51) Int. Cl.: C07C 211/56, C07C 209/00

(54) **Verfahren zur Herstellung von 4,4'-Dinitrodiphenylamin**
Process for preparing 4,4'-dinitrodiphenyl amine
Procédé de préparation de dinitro-4,4' diphénylamine

(30) Priorität: 18.11.1988 DE 3839004
(43) Veröffentlichungstag der Anmeldung: 23.05.1990
(73) Patentinhaber: HOECHST AKTIENGESELLSCHAFT, 65926 Frankfurt am Main (DE)
(72) Erfinder: Tronich, Wolfgang, Dr., D-6239 Eppstein/Taunus (DE); Hess, Peter, Dr., D-6238 Hofheim am Taunus (DE)

(56) Entgegenhaltungen:
- AT-B- 315 819
- DE-B- 1 090 225
- FR-A- 2 409 979

## Beschreibung

Die Erfindung betrifft ein gegenüber dem Stand der Technik verbessertes Verfahren zur Herstellung von 4,4′-Dinitrodiphenylamin.

4,4′-Dinitrodiphenylamin kann nach bekannten Verfahren in 4,4′-Diaminodiphenylamin überführt werden, welches als Zwischenprodukt zur Herstellung von Farbstoffen, beispielsweise als Vorstufe für Colour Index Azoic Diazocomponent 109, oder zur Herstellung von Antioxidationsmitteln für Kautschuk, Öle und Benzin (DE-PS 1 090 225) Verwendung findet.

Zur Herstellung von 4,4′-Dinitrodiphenylamin sind bisher mehrere Verfahren bekannt geworden:
In der FR-A-2 409 979 ist ein Verfahren zur Herstellung von Nitrodiarylaminen durch Umsetzung eines Alkalisalzes eines aromatischen Formylamins mit einem Nitrohalogenaromaten beschrieben.

4,4′-Dinitrodiphenylamin läßt sich durch Dinitrierung von N-Acetyl-diphenylamin und anschließende Entacetylierung erhalten. Ein wesentlicher Nachteil dieses Verfahrens besteht darin, daß die Nitrierung nicht einheitlich verläuft und die gebildeten isomeren Nitrodiphenylamin-Verbindungen durch mehrmaliges Umkristallisieren aus Alkohol sowohl auf der Stufe der Acetylverbindung als auch nach der Verseifung der Acetylverbindung auf der Stufe des freien Amins entfernt werden müssen (Z. Angew. Chemie 12, 1051).

Ein demgegenüber verbessertes Verfahren zur isomerenfreien Herstellung von 4,4′-Dinitrodiphenylamin besteht gemäß der DE-PS 1 090 225 darin, daß man 4-Chlor-nitrobenzol beispielsweise in Gegenwart von Dimethylformamid und von Kupfersalz-Katalysatoren mit 4-Nitroanilin umsetzt. Auch dieses Verfahren ist jedoch trotz einer gewissen Verbesserung nach wie vor mit wesentlichen Nachteilen behaftet.

So erfordert die notwendige hohe Reaktionstemperatur von ca. 200°C bei der technischen Ausübung des Verfahrens aufwendige Heizsysteme. Zum anderen müssen aus ökologischen Gründen die als Katalysatoren benötigten Kupferverbindungen durch aufwendige Abtrennverfahren vollständig aus den Produktionsmutterlaugen entfernt werden und schließlich sind diese Herstellverfahren an die Verwendung von 4-Nitroanilin gebunden, welches seinerseits zunächst durch Umsetzung von 4-Chloranilin mit Ammoniak hergestellt werden muß.

Es bestand somit nach wie vor ein Bedürfnis nach einem einfachen, ökologisch unbedenklichen Verfahren zur Herstellung von 4,4′-Dinitrodiphenylamin.

Es wurde nun gefunden, daß man 4,4′-Dinitrodiphenylamin in einfacher Weise herstellen kann, indem man 1 Mol 4-Halogennitrobenzol mit etwa 1 bis etwa 3 Mol, vorzugsweise etwa 1,5 bis etwa 2 Mol eines Alkalimetallcyanats in Gegenwart von etwa 0,5 bis etwa 5 Mol, vorzugsweise etwa 1 bis etwa 2 Mol Wasser in Dimethylsulfoxid bei Temperaturen von 150 bis 170°C, vorzugsweise 160 bis 165°C, umsetzt.

Als 4-Halogennitrobenzol kommt in erster Linie das 4-Chlornitrobenzol in Betracht.

Als Alkalimetallcyanat kommt Natrium- oder Kaliumcyanat, vorzugsweise Kaliumcyanat in Frage.

Die Menge des Reaktionsmediums Dimethylsulfoxid unterliegt an sich keiner besonderen Begrenzung. Sie soll im allgemeinen jedoch ausreichend sein, um das Reaktionsgemisch rührfähig zu halten.

Die Reaktionsdauer liegt, abhängig von der Reaktionstemperatur, zwischen 15 und 24 Stunden; sie beträgt bei der bevorzugten Reaktionstemperatur insbesondere 16 bis 20 Stunden.

Die Reaktion kann prinzipiell auch in einem geschlossenen Reaktor unter dem sich bei der Reaktionstemperatur einstellenden Überdruck durchgeführt werden.

Die Aufarbeitung erfolgt im allgemeinen durch Verdünnen der Reaktionsmischung mit Wasser. In einer bevorzugten Ausführungsform der Aufarbeitung wird das Reaktionsgemisch in die bis zu 20-fache Gewichtsmenge Wasser eingetragen und das ausgefallene Reaktionsprodukt isoliert. Aus der angefallenen Mutterlauge kann das Dimethylsulfoxid nach üblichen Verfahren, beispielsweise durch fraktionierte Destillation, zurückgewonnen werden.

Das erhaltene 4,4′-Dinitrodiphenylamin wird im allgemeinen vor seiner weiteren Verwendung einem einfachen Reinigungsprozeß unterworfen, der beispielsweise darin besteht, daß das Rohprodukt durch kurze Behandlung mit Wasserdampf von restlichem 4-Nitrochlorbenzol befreit und anschließend mit verdünnter Säure, z.B. Mineralsäure, behandelt wird. Dies kann insbesondere dadurch erfolgen, daß das erhaltene Rohprodukt mit wäßriger Mineralsäure, vorzugsweise wäßriger Salzsäure, ausgerührt und anschließend isoliert wird. Dadurch werden Anteile an ebenfalls gebildetem 4-Amino-nitrobenzol aus dem Produkt herausgelöst. (4-Amino-nitrobenzol kann anschließend aus dem wäßrig-mineralsauren Extrakt durch Zusatz von wäßrigem Alkalihydroxid ausgefällt und anschließend ebenfalls isoliert werden.)

Das erfindungsgemäße Verfahren wird durch die nachstehenden Beispiele näher erläutert, ohne darauf beschränkt zu werden.

### Beispiel 1

79 Gewichtsteile (0,5 Mol) 4-Nitrochlorbenzol werden unter Rühren mit 82 Gewichtsteilen (1,0 Mol) Kaliumcyanat und 18 Gewichtsteilen (1,0 Mol) Wasser in 200 Gewichtsteilen Dimethylsulfoxid 20 Stunden auf 160 bis 165°C erhitzt. Anschließend rührt man die erkaltete Reaktionsmischung in Wasser ein und isoliert das ausgefallene Reaktionsprodukt durch Filtration. Das Feuchtprodukt wird einer Behandlung mit Wasserdampf unterzogen, wobei ca. 2 Gewichtsteile 4-Nitrochlorbenzol abdestillieren. Der Rückstand wird bei ca. 40°C mit wäßriger Salzsäure versetzt, kurz nachgerührt, abgesaugt und getrocknet.

Man erhält 46,5 Gewichtsteile (0,18 Mol) 4,4′-Dinitrodiphenylamin, entsprechend einer Ausbeute von 72 % der Theorie (Fp.: 206 bis 208°C).

Das Filtrat wird durch Zusatz von wäßriger Natronlauge auf pH 7 bis 8 gestellt, Das ausgeschiedene 4-Nitroanilin wird abfiltriert und getrocknet. Man erhält ca. 10 Gewichtsteile 4-Nitroanilin.

Verwendet man an Stelle von 1 Mol Kaliumcyanat 1 Mol Natriumcyanat, so werden deutlich niedrigere Ausbeuten an 4,4′-Dinitro-diphenylamin erhalten.

### Beispiel 2

Es wird gemäß Beispiel 1 gearbeitet mit dem Unterschied, daß pro 79 Gewichtsteile (0,5 Mol) 4-Nitrochlorbenzol 62 Gewichtsteile (0,75 Mol) Kaliumcyanat und 14 Gewichtsteile (0,75 Mol) Wasser eingesetzt werden. Man erhält nach dem Aufarbeiten gemäß Beispiel 1 40 Gewichtsteile (0,15 Mol) 4,4′-Dinitrodiphenylamin, entsprechend einer Ausbeute von 60 % der Theorie (Fp.: 204 bis 206°C).

### Beispiel 3

Es wird gemäß Beispiel 1 gearbeitet mit dem Unterschied, daß pro 79 Gewichtsteile (0,5 Mol) 4-Nitrochlorbenzol 82 Gewichtsteile (1,0 Mol) Kaliumcyanat und 9 Gewichtsteile (0,5 Mol) Wasser eingesetzt werden. Das Ergebnis entspricht dem von Beispiel 1.

### Beispiel 4 (Katalytische Reduktion von 4,4′-Dinitrodiphenylamin)

52 Gewichtsteile 4,4′-Dinitrodiphenylamin (0,2 Mol) werden in 250 Gewichtsteilen Methanol und in Gegenwart von 4 Gewichtsteilen Aktivkohle sowie 2 Gewichtsteilen Dinatriumhydrogenphosphat an 6 Gewichtsteilen Nickel-Katalysator 5 %ig auf Aktivkohle (z.B. vom Typ RCH 55/5 der Firma Ruhrchemie AG) innerhalb von 45 Minuten bei einem Wasserstoffdruck von bis zu 40 bar und einer Temperatur bis zu 140°C ansteigend, reduziert. Nach beendeter Reduktion wird noch heiß vom Katalysator abfiltriert und direkt in ca. 400 Gewichtsteile einer ca. 5 %igen wäßrigen Schwefelsäure eingerührt. Man rührt auf ca. 20°C kalt, saugt ab und wäscht mit Wasser nach. Es werden 54 Gewichtsteile 4,4′-Diaminodiphenylaminsulfat erhalten, entsprechend einer Ausbeute von 91 % der Theorie. Das Sulfatsalz läßt sich durch Behandeln mit wäßrigem Alkalimetallhydroxid in das 4,4′-Diaminodiphenylamin überführen.

## Patentansprüche

1. Verfahren zur Herstellung von 4,4′-Dinitrodiphenylamin, dadurch gekennzeichnet, daß man 1 Mol 4-Halogennitrobenzol mit etwa 1 bis etwa 3 Mol eines Alkalimetallcyanats in Gegenwart von etwa 0,5 bis etwa 5 Mol Wasser in Dimethylsulfoxid bei Temperaturen von 150 bis 170° C umsetzt.

2. Verfahren nach Anspruch 1, dadurch gekennzeichnet, daß man 1 Mol 4-Halogennitrobenzol mit etwa 1,5 bis etwa 2 Mol eines Alkalimetallcyanats in Gegenwart von etwa 1 bis etwa 2 Mol Wasser umsetzt.

3. Verfahren nach mindestens einem der Ansprüche 1 und 2, dadurch gekennzeichnet, daß man 4-Chlornitrobenzol umsetzt.

4. Verfahren nach mindestens einem der Ansprüche 1 bis 3, dadurch gekennzeichnet, daß man mit Kaliumcyanat umsetzt.

5. Verfahren nach mindestens einem der Ansprüche 1 bis 4, dadurch gekennzeichnet, daß man bei Temperaturen von 160 bis 165°C umsetzt.

6. Verfahren nach mindestens einem der Ansprüche 1 bis 5, dadurch gekennzeichnet, daß man in mindestens so viel Dimethylsulfoxid umsetzt, daß die Reaktionsmischung rührfähig bleibt.

## Claims

1. A process for the preparation of 4,4′-dinitrodiphenylamine, which comprises reacting 1 mole of 4-halonitrobenzene with about 1 to about 3 moles of an alkali metal cyanate in the presence of about 0.5 to about 5 moles of water in dimethyl sulfoxide at temperatures of 150 to 170°C.

2. A process as claimed in claim 1, wherein 1 mole of 4-halonitrobenzene is reacted with about 1.5 to about 2 moles of an alkali metal cyanate in the presence of about 1 to about 2 moles of water.

3. A process as claimed in at least one of claims 1 and 2, wherein 4-chloronitrobenzene is reacted.

4. A process as claimed in at least one of claims 1 to 3,wherein the reaction is carried out with potassium cyanate.

5. A process as claimed in at least one of claims 1 to 4, wherein the reaction is carried out at temperatures of 160 to 165°C.

6. A process as claimed in at least one of claims 1 to 5, wherein the reaction is carried out in an amount of dimethyl sulfoxide which is at least such that the reaction mixture remains stirrable.

## Revendications

1. Procédé de préparation de 4,4′-dinitrodiphénylamine, procédé caractérisé ce que l'on fait réagir 1 mol d'un 4-halogénonitrobenzène avec environ 1 à 3 mol d'un cyanate de métal alcalin en présence d'environ 0,5 à 5 mol d'eau dans du diméthylsulfoxyde, à des températures de 150 à 170°C.

2. Procédé selon la revendication 1, caractérisé en ce que l'on fait réagir 1 mol du 4-halogénonitrobenzène avec environ 1,5 à 2 mol du cyanate de métal alcalin en présence d'environ 1 à 2 mol d'eau.

3. Procédé selon la revendication 1 ou 2, caractérisé en ce que l'on fait réagir du 4-chloronitrobenzène.

4. Procédé selon une ou plusieurs des revendications 1 à 3, caractérisé en ce que l'on utilise du cyanate de potassium.

5. Procédé selon une ou plusieurs des revendications 1 à 4, caractérisé en ce que l'on opère à des températures de 160 à 165°C.

6. Procédé selon une ou plusieurs des revendications 1 à 5, caractérisé en ce que l'on opère dans une quantité de diméthylsulfoxyde au moins suffisante pour que le mélange de réaction puisse être toujours agité.
